Europäisches Patentamt

European Patent Office

Office européen des brevets

Publication number: **0 284 965**

A2

# EUROPEAN PATENT APPLICATION

Application number: 88104588.4

Int. Cl.⁴ **A61K 37/02** , A61K 35/64

Date of filing: 22.03.88

Priority: 30.03.87 JP 77154/87

Date of publication of application:
05.10.88 Bulletin 88/40

Designated Contracting States:
CH DE FR GB IT LI

Applicant: SANWA KAGAKU KENKYUSHO CO., LTD.
No. 35, Higashi-sotobori-cho
Higashi-ku Nagoya-shi Aichi-ken(JP)

Inventor: Natori, Shunji
2208, 19, Fukawa, Tone-machi
Kita-soma-gun Ibaragi-ken(JP)

Representative: Wächtershäuser, Günter, Dr.
Tal 29
D-8000 München 2(DE)

Antibacterial polypeptide.

An antibacterial polypeptide is disclosed, which is immunologically produced by _Sarcophaga peregrina_, when the insect is injured in its body wall. The polypeptide has molecular weight of about 7000.

EP 0 284 965 A2

## Antibacterial polypeptide

The present invention relates to a novel antibacterial polypeptide which is obtained from a body fluid of an insect and more particularly from flesh fly (Sarcophaga peregrina).

It has been known that a certain antibacterial substance will appear in a body fluid, when a vaccine is inoculated to an invertebrate such as insecta [ "Eur. J. Biochem." Vol. 106, page 7 (1980)].

The present inventor has also found that the Sarcophaga peregrina produces a certain antibacterial polypeptide in its body fluid, when a larva of the insect is injured in its body wall, the polypeptide being separated and purified to investigate its physicochemical properties [see Jap. Pat. Nos. 59 - 13730 (A) published January 24, 1984 and 61 -122299 (A) published June 10, 1986].

Since the polypeptide produced by the insect shows a wide antibacterial spectrum, almost no toxicity and is one of proteins, the substance has been expected as one of edible antibiotics but a yield thereof is not so high.

A basic object of the invention, therefore, lies in investigating biologically active substances to be produced by the Sarcophaga peregrina and other than said antibacterial peptide disclosedin said Japanese patent literatures to increase a utilization efficiency of the insect.

According to the invention, the object is attained by an antibacterial polypeptide obtained from Sarcophaga peregrina injured in its body wall and having

a) a molecular weight of about 7000, when it measured by SDS polyacryloamide-gel electrophoresis, and

b) an amino acid composition of

| | | |
|---|---|---|
| Asp + Asn | 12.6 (mol %) | |
| Thr | 3.8 | |
| Ser | 7.7 | |
| Glu + Gln | 9.9 | |
| Pro | 8.1 | |
| Gly | 15.9 | |
| Ala | 2.1 | |
| Cys | 0 | |
| Val | 4.6 | |
| Met | 0 | |
| Ile | 1.3 | |
| Leu | 3.9 | |
| Tyr | 5.1 | |
| Phe | 5.7 | |
| Lys | 6.8 | |
| His | 3.1 | |
| Arg | 9.3 | |
| Trp | 0. | |

The polypeptide according to the invention shows an antibacterial activity as compared with that in the previous polypeptide (molecular weight of about 4000) disclosed in said Japanese patent literatures, has a quite low toxicity, and is excellent in thermal stability.

The polypeptide (molecular weight of about 7000) according to the invention can be obtained in a manner similar to that as disclosed in said literatures, namely by rearing pupae or larvae of Sarcophaga peregrina over a certain period of time, after giving an injury in its body wall, taking out a body fluid or homogenizing a whole body and removing solid substances to obtain a liquid component as raw material, extracting and fractionating the liquid through ion-exchange chlomatography and HPLC reverse-phase chlomatography to collect fractions with an antibacterial activity.

The ground that the pupae or larvae are selected for the Sarcophaga peregrina as the producing insect lies in such a known fact that a certain antibacterial polypeptide is also produced, when an imago of the insect is injured in its body, but its antibacterial activity is lower than that produced by the pupa or larva thereof. The rearing period of time from the time, when the insect is injured, to the other time, when the body fluid is taken out or the whole body of the insect is homogenized has been set to give a sufficient period of time for production of the desired antibacterial polypeptide. It is preferable for the rearing period of time to set as, for instance, 24 to48 hours, since reduction of antibacterial activity of the produced polypeptide will occur, when the rearing period of time is too long.

The ion-exchange chlomatography is carried out in multi-stage manner. Among fractions obtained in the last stage, those showing relatively high antibacterial activity are collected and purified through the HPLC reverse-phase chlomatography to give the antibacterial polypeptide having molecular weight of about 4000 and as disclosed in said Jap. Pat. Nos. 59 - 13730 (A) and 61 - 122299 (A), respectively. While, other fractions showing relatively low antibacterial activity in the final stage are collected and purified in a similar manner to unexpectedly find out that the antibacterial polypeptide according to the present invention and having molecular weight of about 7000 can be obtained.

The invention will now be further explained with reference to an Example for obtaining the antibacterial polypeptide as referred to, as well as Test Example, which will refer also to drawings, wherein

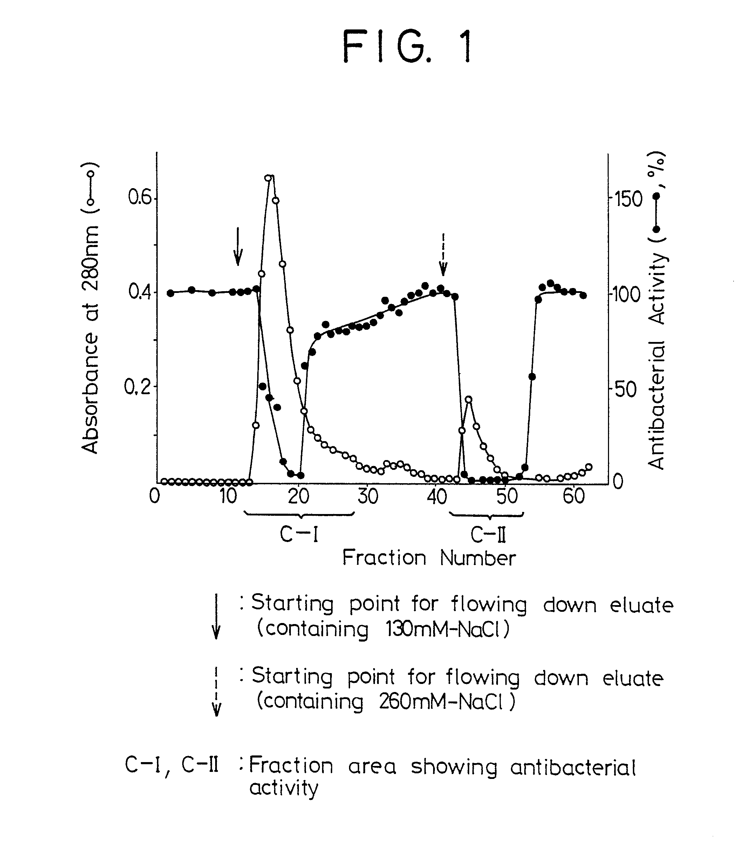

Fig. 1 is a graph showing relations of each fraction obtained by a linear gradient elution with use of NaCl-containing phosphate buffer in second CM cellurose column chlomatography for extraction steps of an antibacterial polypeptide according to the invention, an absorbance of the fraction and an antibacterial activity thereof;

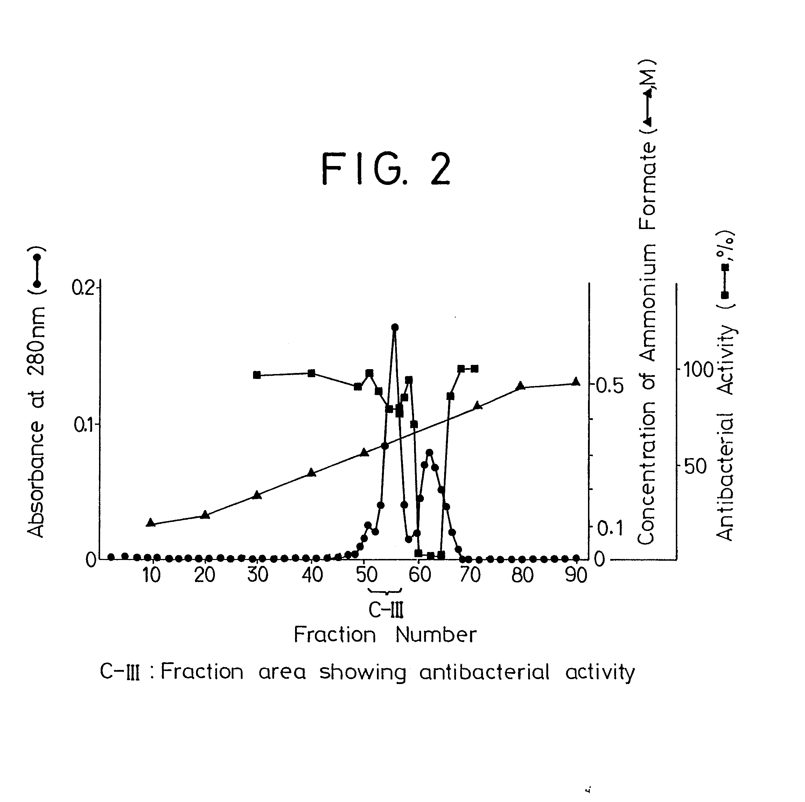

Fig. 2 is a graph similar to that of Fig. 1 but showing relations of each fraction obtained by a linear gradient elution with use of ammonium formate solution on a fraction area C-II in Fig. 1, an absorbance of the fraction and an antibacterial activity thereof;

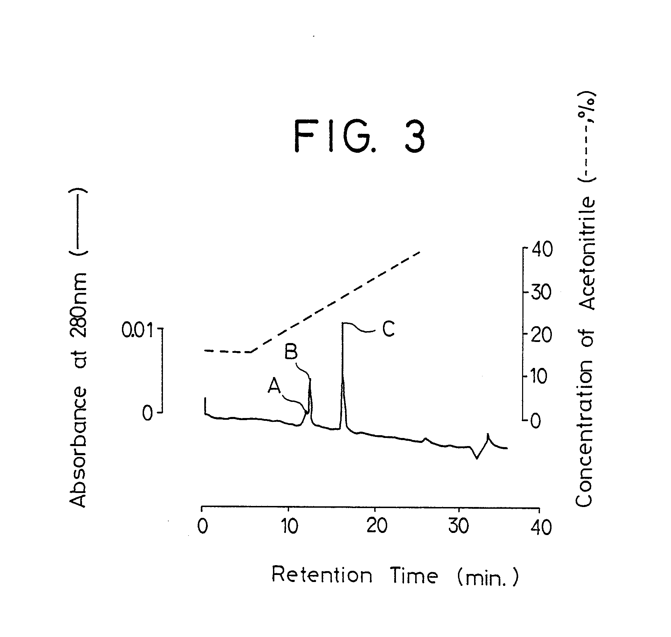

Fig. 3 is a graph similar to those in Figs 1 and 2 but showing relations of each fraction obtained by carrying out HPLC reverse-phase chlomatography on a fraction area C-III in Fig. 2 and gradiently eluting with use of acetonitrile, and absorbance of the fraction and an antibacterial activity thereof;

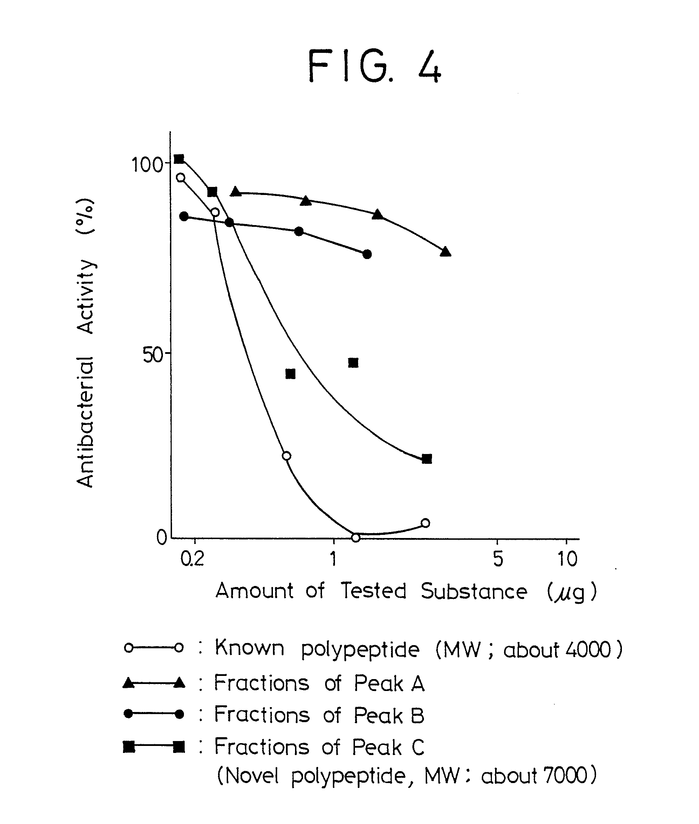

Fig. 4 is a graph showing an anti-virus activity of fraction areas corresponding to peaks A, B and C in Fig. 3 as well as known antibacterial polypeptide having molecular weight of about 4000; and

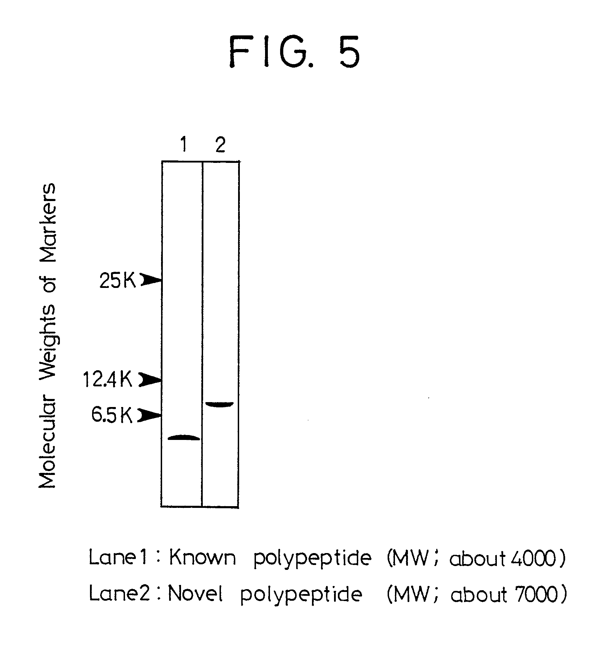

Fig. 5 is an illustration showing cataphoretic pattern, when a molecular weight of the antibacterial polypeptide according to the invention was determined by SDS polyacrylamide-gel electrophoresis, together with the known antibacterial polypeptide, as control or reference and various molecular weight markers.

## Example

### 1) Preparation of raw material

A body wall of each larva of Sarcophaga peregrina (third-instar larvae, namely those matured or mellowed by repeating an ecdysis in 3 times) wasinjured with use of an injection needle and then reared for 24 to 28 hours under room temperature. A tail end of the larvae was cut with scissors to squeeze out a body fluid on an ice-cooled petri dish. The body fluid was subjected to a centrifugal treatment (200 x g, 10 minutes) to remove solid substances and obtain a supernatant which will be employed as raw material.

For a reserve, the supernatant is freezed - 80°C.

### 2) Pretreatments (Multi-stage ion-exchange chlomatography)

#### a) First CM cellulose column chlomatography

To 30ml of the raw material body fluid, 120ml of 10mM-phosphate buffer were added to adjust pH of the solution to 6.0. The resulting solution was applied to a CM cellulose column (3.4 x 20.0cm) which was then washed with a fresh buffer solution same with the above. To the column, 250mM-NaCl containing phosphate buffer (pH 6.0) was passed to collect each fraction by 5ml. An antibacterial activity according to the method by Okada et al and using an Escherichia coli (K12 594 strain) [ "Biochem. J." Vol. 211, pages 724 to 734 (1983)] as well as absorbances at 250 and 650nm were measured on each fraction to identify fraction(s) having antibacterial activity.

#### b) Sephadex G-50 column chromatography

The antibacterial fractions confirmed in said Item a were combined and heated at 100 °C for 10 minutes and then centrifuged to remove a precipitate formed therein. The resulting supernatant was concentrated through a ultrafiltration. The concentrate was applied to Sephadex G-50 column (1.5 x 60.0cm) and eluted with 130mM-NaCl containing phosphate buffer (pH 6.0) to collect fractions by each 2ml. An antibacterial activity and an absorbance at 280nm of each fraction were measured in the manner similar to that in said Item a.

The results obtained in said Items a and b are substantially same with those as disclosed in the aforementioned Jap. Pat. Nos. 59 -13730 (A) and 61 - 122299 (A).

#### c) Second CM cellulose column chlomatography

The fraction area which shows highest antibacterial activity among the fractions obtained through said Item b was corrected and diluted with use of 10mM-phosphate buffer to make its volume in 5 times. The resulting solution was applied again to CM cellulose column (2.0 x 4.0cm) and an adsorptive was eluted with a linear gradient of 25mM to 100mM-NaCl containing phosphate buffer to collect fractions by each 3ml. An antibacterial activity and an absorbance at 280nm of each fraction were measured as the manner similar to that stated in said Itema to obtain results shown in Fig. 1.

#### d) CM Sepharose column chlomatography

As apparently seen from Fig. 1, there were two fraction areas (C-I and C-II) which show antibacterial activity. One of the fractiuon areas (C-I fraction area) is that obtained some time after begining the flow-down of 130mM-NaCl containing phosphate buffer and shows relatively high antibacterial activity. If the fractions in this area are further treated and purified in a manner similar to that to be described later, an antibacterial polypeptide having molecular weight of about 4000 can be obtained, as disclosed in the aforesaid Jap. Pat. Nos. 59 - 13730 (A) and 61 - 122299 (A).

While, in this Example, fractions (Fraction Nos. 44 to 53, 30ml in total) in another fraction area (C-II area) which are obtained some time after begining the flow-down of 260mM-NaCl containing phosphate buffer and show relatively low antibacterial activity were recovered and diluted with 250ml of ammonium formate solution.

A CM Sepharose column (0.8 x 20cm) was equilibrated with 10mM ammonium formate solution and the diluted solution was applied to the column to adsorb a protein component therein, which was eluted with 60ml of a linear gradient of 0.1M to 0.5M-ammonium formate solution and fraction was collected by each 1ml. An antibacterial activity and an absorbance at 280nm of each fraction were measured in a manner similar to that in said Item a. Results are shown in Fig. 2.

3) Purifying treatment (Purification of antibacterial polypeptide)

As apparently seen from Fig. 2, there were also two fraction areas showing an antibacterial activity. Among them, the first fraction area (C-III area, Fraction Nos. 52 to 56, 5ml in total) showing relatively high antibacterial activity was recovered and concentrated. The concentrate was fractionated with a reverse-phase HPLC (Synchropack RPP-C18 column) chlomatography under following conditions.

Reagent A : 0.05% Trifluoroacetate/water,

Reagent B : 0.05% Trifluoroacetate/99% Acetonitrile,

Gradient : Linear gradient with use of 15% Reagent B in Reagent A and 50% Reagent B in Reagent A,

Flow velocity : 2ml/min.

Results are shown in Fig. 3. It has been found that a value of absorbance reduces and tends into negative value, as a concentration of acetonitrile increases and that there are fractions showing peaks (A, B and C) in absorbance.

Therefore, each of fraction areas was recovered and an antibacterial activity thereof was measured in a manner similar to that in said Item a. Results are shown in Fig. 4. As seen apparently from the Figure, the fractions corresponding to the peak C only show the antibacterial activity.

In Fig. 4, there is also shown an antibacterial activity of the known antibacterial polypeptide disclosed in Jap. Pat. Nos. 59 - 13730 (A) and 61 - 122299 (A) and having molecular weight of about 7000, as a reference. Comparing the data thereof with those in the antibacterial polypeptide (fractions corresponding to peak C) according to the invention, the latter is somewhat weak.

Test Example 1 (Measurement of molecular weight)

A molecular weight of the antibacterial polypeptide (fractions corresponding to peak C) obtained by said Example, as a test sample was measured by 15% SDS polyacrylamide-gel electrophoresis. As a control sample, the antibacterial polypeptide as disclosed in Jap. Pat. Nos. 59 - 13730 (A) and 61 - 122299 (A) was employed, which has molecular weight of about 4000. These test and control samples were treated with 1% SDS and 2% β-mercaptoethanol. Further, chymotrypsinogen (MW : 25000), cytochrome C (MW : 12400) and aprotinin (MW : 6500) were selected as molecular weight markers.

Results are shown in Fig. 5. With reference to cataphoretic positions of the molecular weight markers, it is apparent that the control sample (Lane 1) and test sample (Lane 2) have molecular weight of about 4000 and about 7000, respectively and thus the both antibacterial polypeptides are different with each other.

Test Example 2 (Amino acid analysis)

25μg of the antibacterial polypeptide (fractions corresponding to peak C) obtained by said Example were treated with 6N-HCl for 12 hours at 120°C to cause a hydrolysis thereof. An amino acid analysis of the resulting hydrolysate was carried out with use of an automatic amino acid analizer (Type 835, manufactured and marketed by Hitachi Ltd., Japan). As a result, it was found that the antibacterial polypeptide has following amino acid composition.

| Asp + Asn | 12.6 (mol %) |
| Thr | 3.8 |
| Ser | 7.7 |
| Glu + Gln | 9.9 |
| Pro | 8.1 |
| Gly | 15.9 |
| Ala | 2.1 |
| Cys | 0 |
| Val | 4.6 |
| Met | 0 |
| Ile | 1.3 |
| Leu | 3.9 |
| Tyr | 5.1 |
| Phe | 5.7 |

Lys    6.8
His    3.1
Arg    9.3
Trp    0.

Test Example (Toxicity)

The antibacterial polypeptide obtained in the Example was dissolved into saline, and the resulting solution was injected subcutaneously or intra peritoneally to BALB/c mice and ICR mice over 10 days in an amount of 100µg/kg, but there was observed neither anaphylactic shock nor necrosis, inflammation or the like, in an autopsy.

Test Example (Thermal stability)

The antibacterial polypeptide obtained in the Example was dissolved into saline, and the resulting solution was heated to 100 °C to keep for 20 minutes at the temperature, and then left to stand to allow cooling. An antibacterial activity of the solution was measured in accordance with the method proposed by Okada et al, as in the Example, but there is found not noticeable reduction in the activity.

## Claims

1. An antibacterial polypeptide obtained from Sarcophaga peregrina injured in its body wall and having
    a) a molecular weight of about 7000, when it was measured by SDS polyacrylamide-gel electrophoresis, and
    b) an amino acid composition of

Asp + Asn    12.6 (mol %)
Thr    3.8
Ser    7.7
Glu + Gln    9.9
Pro    8.1
Gly    15.9
Ala    2.1
Cys    0
Val    4.6
Met    0
Ile    1.3
Leu    3.9
Tyr    5.1
Phe    5.7
Lys    6.8
His    3.1
Arg    9.3
Trp    0.

0 284 965

# F I G. 1

↓ : Starting point for flowing down eluate
   (containing 130mM-NaCl)

↓ : Starting point for flowing down eluate
   (containing 260mM-NaCl)

C−I, C−II : Fraction area showing antibacterial
            activity

# FIG. 2

C-III : Fraction area showing antibacterial activity

# FIG. 3

# FIG. 4

o———o : Known polypeptide (MW ; about 4000 )

▲———▲ : Fractions of Peak A

●———● : Fractions of Peak B

■———■ : Fractions of Peak C
(Novel polypeptide, MW : about 7000)

# FIG. 5

Lane 1 : Known polypeptide (MW; about 4000)
Lane 2 : Novel polypeptide (MW; about 7000)